# EUROPEAN PATENT SPECIFICATION

(11) **EP 1 370 263 B1**
(45) Date of publication and mention of the grant of the patent: **18.08.2004**
(21) Application number: 02716990.3
(22) Date of filing: 25.03.2002
(51) Int. Cl.: A61K 31/495, A61K 31/65, A61K 31/43, A61K 31/48, A61P 31/04

(54) **ANTIBIOTIC PHARMACEUTICAL COMPOSITION WITH LYSERGOL AS BIO-ENHANCER AND METHOD OF TREATMENT**
ANTIBIOTISCHE PHARMAZEUTISCHE FORMULIERUNG MIT LYSERGOL ZUR VERSTÄRKUNG DER WIRKSAMKEIT UND BEHANDLUNGSMETHODE
COMPOSITION PHARMACEUTIQUE ANTIBIOTIQUE CONTENANT DU LYSERGOL EN TANT QUE BIO-ACTIVATEUR ET METHODE DE TRAITEMENT

(43) Date of publication of application: 17.12.2003
(73) Proprietor: Council of Scientific and Industrial Research, New Delhi 110 001 (IN)
(72) Inventor: Khanuja, Suman Preet Singh, Central Inst. Med., Uttar Pradesh (IN); Arya, Jai Shankar, Central Inst. of Med. Aromatic, Uttar Pradesh (IN); Srivastava, Santosh Kumar, Central Inst. Med., Uttar Pradesh (IN); Shasany, Ajit Kumar, Central Inst. of Med., Uttar Pradesh (IN); Kumar, Tiruppadiripuliyur R., Central Ins. Med., Uttar Pradesh (IN); Darokar, Mahendra Pandurang, Central Inst. Med., Uttar Pradesh (IN); Kumar, Sushil, Central Inst. Med. and Aromatic, Uttar Pradesh (IN)
(74) Representative: Hayes, Adrian Chetwynd
(86) International application number: PCT/IB2002/001125
(87) International publication number: WO 2003/080059

(56) References cited:
- US-A- 4 618 581
- EICH E. ET AL: "Antimicrobial activity of clavines." ARZNEIMITTEL-FORSCHUNG/DRUG RESEARCH, (1985) 35/12 (1760-1762). CODEN: ARZNAD, XP001080009

## Description

### TECHNICAL FIELD

The invention relates to a synergistic antibiotic pharmaceutical composition with lysergol as bioactive enhancer and bioavailability facilitator for broad-spectrum antibiotics. The present invention has direct implication in reducing the dosage of antibiotics while increasing the efficiency of absorption of nutritional elements. The present invention also provides a method of treatment for bacterial infections.

### BACKGROUND ART

The consumption of antibiotics and drugs by man is increasing at an alarming rate. Out of the total drugs and chemicals, 20%-50% of that use is unnecessary depending on the class of antibiotic. In addition, indiscriminate use of antibiotics promotes antibiotic resistance leading to multiple drug resistance and makes it difficult to control the diseases. Really speaking, the infected individuals consume much more amount of antibiotics in the given dosage that is actually required to control a given population of parasite in the body. This may be due to (i) reduced absorption in the gut membrane when taken orally (ii) restrictive uptake by the target microbe or (iii) operation of efflux pump leading to indiscriminate extrusion of the antibiotics or therapeutic molecules. So the major amount of the drugs we apply are wasted and only a minor percentage is being targeted to the infective microbes.

In addition, the unutilized drug / antibiotic amount remains as a load in the body and environment acting as a selection pressure facilitating emergence of drug resistance in parasites and their predominance, ultimately leading to failure of antibiotics against resistant infections. This also is responsible for side effects, illness and reduction in life expectancy. One of the ways, which has been feasible to reduce drug dosage, has been synergism between two therapeutic agents. However, if both have the antibiotic property, still the problem of continued selection pressure on microbes is likely to continue. So, we thought of searching only those molecules, which by them are not microbicidal but when present with a drug or active molecule, enhance its activity and availability (bioenhancers). This way these molecules by their presence will not exert any selection pressure for mutants to emerge resistant against them and on the other hand could reduce the dosage of antibiotics or drugs so that their ill effects are minimized and the resistance development process will be substantially delayed ultimately leading to enhanced life-span of the novel and existing antibiotics. Such drug / molecule facilitators should have novel properties like non-toxic to human, animal or plants, should be effective at a very low concentration in a combination, should be easy to formulate and most importantly enhance uptake/absorption and activity of the drug molecules. This can lead in developing judicious and strategic concentrations of antibiotics with specific bioenhancers to improve availability of the drug right up to the target for effectively controlling the infectious organisms. The present invention was the result of planned experiments to provide a plant compound 'Lysergol' with novel properties for improving activity and bioavailability of antibiotics, drugs and other molecules in different formulations. The bioavailability enhancement of antibiotic effect is relevant to human, plant as well as animal health and thus the compositions and methods of the invention are also intended to be used in agriculture and veterinary practice.

Use of ayurvedic preparation "trikatu" dates back to the period between the seventh century B.C. and the sixth century A.D, which is a Sanskrit, word meaning three acrids. It refers to a combination of black pepper (*Piper nigrum* Linn.), long pepper (*Piper longum* Linn.) and ginger (*Zingiber officinale* Rosc.). It is believed that the use of "trikatu", and its constituents individually as well as collectively, enhances the bioavailability of a number of drugs. In specific studies carried out on animals as well as human volunteers, it was noted that the active component responsible for the increase in bioavailability of various drugs was piperine (United States Patent 5,616,593 and 5,972,382). Till today thus, the known bio-availability enhancer documented is piperine and a series of inventions related to this compound have been described in the following prior arts. Though the compound piperine has been reported to be enhancing the bioavailability of drugs, nutrients and vitamins, still a proper formulation for the combination is yet to come to the market.

The present invention is to obtain a molecule with bioenhancing action of higher potency.

Thus a large numbers of the available extracts and known compounds are screened in the laboratory, particularly those by themselves possessing no antibacterial property. After extensive experimentation, it has been found that a plant compound lysergol enhanced the killing activities of different antibiotics on bacteria. The compound is isolated from genera of lower fungi: *Claviceps, Penicillium* and *Rhizopus*. From higher plants like *Rivea corymbosa, Ipomoea violacea* and *Ipomoea muricata* the compound is also isolated and well-defined isolation protocols are already available. The seeds of *Ipomoea muricata* are commonly known as 'Kaladana' in trade and are being used as purgative in Pakistan and India. The seeds are a good source of clavine alkaloids. The seeds are reported to contain 0.49% of total alkaloid, out of which lysergol constitutes 53% and chanoclavine 37%. Lysergol is used as hypotensive, psychotrophic, analgesic, immunostimulant, analeptic and uterus and intestine stimulating drug. Also, the compound is available commercially (Sigma Chemicals, USA).

The compound lysergol is chemically known as 9,10-Didehydro-6-methylergoline-8-□-methanol.

Ergotamine and all compounds either structurally and/or pharmacologically similar to it like lysergol are 5HT agonist vasoactive agents (United States Patent 6,077,539) that means ensure normal blood flow in blood vessels in a therapeutically effective amount.

This compound also is reportedly psychoactive causing nausea, which may be experienced during first hour. This compound also has hallucination and anti-tension properties.

Great emphasis now is being laid towards quality assurance of crude drugs from plants sources widely used in the Indian system of medicine. The scientific study of traditional medicines, derivation of drugs through bioprospection and systematic conservation, domestication and cultivation of the concerned medicinal plants has assumed great importance in the present day context when more and more people prefer safe and effective medicines at affordable price for curing their ailments. The present invention enlarges the scope and use of the natural plant compound lysergol in therapeutical applications.

### OBJECTS OF THE INVENTION

Main object of the present invention is to provide a non-toxic bioenhancer lysergol to be used in an antibiotic pharmaceutical composition.

Another object of the present invention is to provide a synergistic antibiotic pharmaceutical composition for the treatment of bacterial infections.

Yet another object of the present invention is to provide an antibiotic pharmaceutical composition having reduced concentration of antibiotic compounds.

Further object of the present invention is to provide an antibiotic pharmaceutical composition to prevent antibiotic drug resistance.

### SUMMARY OF THE IVNENTION

The invention provides a synergistic pharmaceutical composition with lysergol as bioenhancer of antibiotic action on the target. The molecule of invention helps in the absorption of antibiotics across the cell membrane in animal cells for action against gram positive and negative bacteria. The present invention also provides a method of treatment for bacterial infections.

### DETAILED DESCRIPTION OF THE INVENTION

Accordingly, the present invention provides a synergistic antibiotic pharmaceutical composition having enhanced bioactivity, said composition comprising:
(a) an antibiotic compound 0.2-20 µg/ml;
(b) an effective amount lysergol 1-10µg/ml or 2-10µg/ml; and
(c) optionally pharmaceutically acceptable additives.

An embodiment of the present invention, wherein the antibiotic is selected from the group consisting of rifampicin, tetracycline and ampicillin.

Yet another embodiment of the present invention, wherein the preferable dosage of lysergol is 10µg/ml.

Still another embodiment of the present invention, wherein the enhanced activity of antimicrobial effect is in the range of 2-12 folds.

Yet another embodiment of the present invention, wherein said composition is effective against broad-spectrum microbes both gram positive and negative, selected from the group consisting *E*.*coli*, *Bacillus subtilis and Mycobacterium smegmatis* and other similar microbes.

Further embodiment of the present invention, wherein lysergol is isolated from a genera of lower fungi consisting of Claviceps, Pencillium and Rhizopus and from the plants selected from Rivea corymbosa and ipomoea violace.

Yet another embodiment of the present invention, wherein lysergol enhances the transport of antibiotics across the intestinal gut and cell membrane for better efficacy on the target site.

Still another embodiment of the present invention, wherein the reduced dosage of antibiotics and the enhanced bioactivity of the composition reduces the ill effects of antibiotics.

Yet another embodiment of the present invention, wherein the resistance to antibiotics is substantially reduced due to reduced concentration of antibiotics.

The present invention also provides a method of treating bacterial infection, wherein administering to subject an effective amount of synergistic pharmaceutical composition, said composition comprising:
(a) an antibiotic compound;
(b) an effective amount lysergol 1-10µg/ml or 2-10µg/ml; and
(c) optionally pharmaceutically acceptable additives.

An embodiment of the present invention, a method wherein the antibiotic is selected from the group consists of rifampicin, tetracycline and ampicillin.

Yet another embodiment of the present invention, a method wherein the preferable dosage of lysergol is 10µg/ml.

Still another embodiment of the present invention, a method wherein the enhanced activity of antimicrobial effect is in the range of 2-12 folds.

Further embodiment of the present invention a method wherein said composition is effective against broad-spectrum microbes both gram positive and negative, selected from the group consisting *E.coli, Bacillus subtilis and Mycobacterium smegmatis* and other similar microbes.

Yet another embodiment of the present invention, a method wherein lysergol is isolated from genera of lower fungi consisting of Claviceps, Pencillium and Rhizopus and from higher plants selected from Rivea corymbosa and ipomoea violace.

Still another embodiment of the present invention, a method wherein lysergol enhances the transport of antibiotics across the intestinal gut and cell membrane for better efficacy on the target site.

Further embodiment of the present invention, a method wherein the reduced dosage of antibiotics and the enhanced bioactivity of the composition reduces the ill effects of antibiotics.

Still another embodiment of the present invention, a method wherein the resistance to antibiotics is substantially reduced due to reduced concentration of antibiotics.

Yet another embodiment of the present invention, a method wherein the subject is selected from mammals and humans.

The invention is further explained in the form of following embodiments.
1. Assay for bio-enhancement of anti-infective agents
   (a) The minimum inhibitory concentration (MIC) of antibiotic is determined against *Escherichia coli* (ATCC 10536), *Bacillus subtilis* (ATCC 6051) and
      *Mycobacterium smegmatis* (ATCC 14468) in broth and disc diffusion assay.
   (b) The antibiotics at concentrations 1/4, 1/3, 1/2 and equal to MIC are added alone and in combination with the test compound at varying concentrations on disc and in broth to evaluate the comparative inhibition.
   (c) These combinations showing significant advantage or higher activity than antibiotic alone in terms of enhanced inhibition of bacterial growth (large inhibition zone in disc diffusion and effectivity of lower concentration in broth assay) are picked up for future testing.
   (d) In broth assay the activity is quantified by counting number of viable cells in a given treatment and converted in fold enhancement by combination compared to antibiotic / drug alone in the killing percentage of cells.
   (e) The pretreatment assay followed to determine whether the compound is required along with antibiotic to enhance its activity or even its withdrawal after treatment or prior to antibiotic treatment would benefit. For this, the cells are treated with compound for 4 to 8 hours and then washed free of it by centrifugation and washing in sterile water. This is followed by treatment with antibiotic as in steps b to d.

### Process for the isolation of lysergol.

The seeds of *Ipomoea muricata* are powdered and defatted with hexane and then extracted with methyl alcohol. The alcoholic extract is dried and extracted with 5-10% Hcl solution.

The acidic extract is then converted to basified upto pH 9.0 and extracted with chloroform and butanol successively. The crude alkaloid obtained in chloroform and butanol extract is further purified by column chromatography to yield lysergol in maximum yield upto 0.2%.

Bioactivity is experimented with the killing activities of different antibiotics against the bacteria singly and in combination with the test compound Lysergol following the method described above. These experiments are being described in the following examples. When the bacteria are grown in presence of the compound as such no significant killing is observed. In all the experiments the Lysergol concentration is kept at 10 µg/ml, unless it is specifically mentioned.

The invention is further explained in the form of examples.

### Example 1

Lysergol mediated enhancement in the killing action of antibiotics against Gram negative bacterium *Escherichia coli.*

**Table 1:**

| Antibiotics | Concentration µg/ml | Survival fraction of viable cells upon treatment with antibiotic alone | Survival fraction of viable cells upon treatment with antibiotic + lysergol combination | *Fold enhancement in antibiotic activity |
|---|---|---|---|---|
| Rifampicin | 10 | 0.35-0.45 | 0.037-0.058 | 6-12 |
| Rifampicin | 20 | 0.25-0.30 | 0.060-0.083 | 3-5 |

| | | | | |
|---|---|---|---|---|
| *It is calculated as = Survival fraction of viable cells upon treatment with antibiotic and lysergol in combination / Survival fraction of viable cells upon treatment with antibiotic alone | | | | |

### Example 2.

Lysergol mediated enhancement in the killing action of antibiotics against Gram positive bacterium *Bacillus subtilis*.

**Table 2:**

| Antibiotics | Concentration µg/ml | Survival fraction of viable cells upon treatment with antibiotic alone | Survival fraction of viable cells upon treatment with antibiotic + lysergol combination | *Fold enhancement in antibiotic activity |
|---|---|---|---|---|
| Rifampicin | 0.4 | 0.085-0.096 | 0.021-0.028 | 3.0-4.6 |

| | | | | |
|---|---|---|---|---|
| *It is calculated as = Survival fraction of viable cells upon treatment with antibiotic and lysergol in combination / Survival fraction of viable cells upon treatment with antibiotic alone | | | | |

### Example 3

Lysergol mediated enhancement in the killing action of antibiotics against bacterium *Mycobacterium smegmatis*

**Table 3:**

| Antibiotics | Concentration µg/ml | Survival fraction of viable cells upon treatment with antibiotic alone | Survival fraction of viable cells upon treatment with antibiotic + lysergol combination | *Fold enhancement in antibiotic activity |
|---|---|---|---|---|
| Rifampicin | 0.2 | 0.45-0.54 | 0.09-0.10 | 4.5-6.0 |

| | | | | |
|---|---|---|---|---|
| *It is calculated as = Survival fraction of viable cells upon treatment with antibiotic and lysergol in combination / Survival fraction of viable cells upon treatment with antibiotic alone | | | | |

From the above experiments it is deduced that the potency of the antibiotic is increased against both Gram positive and negative bacteria when applied along with the compound lysergol.

### Example 4

Lysergol mediated enhancement in the killing action of antibiotic against bacteria in disc diffusion assays.

**Table 4:**

| Content of left arm | Absorbency at 340 nm (for rifampicin) and 223 nm (for tetracycline) | | | |
|---|---|---|---|---|
| | 0h | 2h | Increase in absorbency | Fold increase |
| Rifampicin | 0.048 | 0.179 | 0.131 | - |
| Rifampicin + lysergol | 0.048 | 0.437 | 0.389 | 2.96 |
| Tetracycline | 0.254 | 0.612 | 0.358 | - |
| Tetracycline + lysergol | 0.254 | 2.422 | 2.168 | 8.53 |

In other observations the compound lysergol enhances the transport of antibiotics e.g. Rifampicin, Tetracycline across the gut as well as artificial membrane. We performed the experiments in U-shaped tubes with joint in between, where the freshly isolated gut membrane is fixed. In control tube only antibiotic solution (4ml @ 1mg/ml solution) is poured in the left arm where as in the right arm only water is poured. In the other tube in addition to the antibiotic solution lysergol (4µg @ 1µg/ml) is added. Then changes in absorbency in the right arm of both the tubes are noted at 340nm (for rifampicin) and 223nm (for tetracycline). The enhancement in transport is approximately 2.96 to 8.53 folds. This in-turn has immense importance for absorption of the drugs, pharmaceuticals, nutraceutical and other related compounds and ions by the cells.

### ADVANTAGES

1. The main advantage of the present invention is the reduction of antibiotic dosage by means of synergistic composition.
2. Reduction in antibiotic dosage resulting in prevention of antibiotic drug resistance.
3. Incorporation of bioactive enhancer in the antibiotic composition, which non-toxic to animals and humans.

## Claims

1. A synergistic antibiotic pharmaceutical composition having enhanced bioactivity in subjects, said composition comprising:
(a) an antibiotic compound;
(b) an effective amount lysergol 1-10 µg/ml; and
(c) optionally pharmaceutically acceptable additives.

2. A pharmaceutical composition according to claim 1, wherein the antibiotic is selected from the group consisting of rifampicin, tetracycline and ampicillin.

3. A pharmaceutical composition according to claim 1, wherein the preferable dosage of lysergol is 10 µg/ml.

4. A pharmaceutical composition according to claim 1, wherein the enhanced activity of antimicrobial effect is in the range of 2-12 folds over antibiotic compounds used singly.

5. A pharmaceutical composition according to claim 1, wherein said composition is effective against broad-spectrum microbes both gram positive and negative, selected from the group consisting *E*. *coli*, *Bacillus subtilis and Mycobacterium smegmatis* and other similar microbes.

6. A pharmaceutical composition according to claim 1, wherein lysergol is isolated from genera of lower fungi consisting of Claviceps, Pencillium and Rhizopus and from the plants selected from Rivea corymbosa and ipomoea violace.

7. A pharmaceutical composition according to claim 1, wherein lysergol enhances the transport of antibiotics across the intestinal gut and cell membrane for better efficacy on the target site.

8. A pharmaceutical composition according to claim 1, wherein the reduced dosage of antibiotics and the enhanced bioactivity of the composition reduces the ill effects of antibiotics.

9. A pharmaceutical composition according to claim 1, wherein the resistance to antibiotic is substantially reduced to reduced concentration of antibiotic.

10. A pharmaceutical composition according to claim 1, wherein the subject is selected from mammals and humans.

11. A composition as claimed in any one of the preceding claims for use as synergistic antibiotic.

## Patentansprüche

1. Synergistische antibiotische pharmazeutische Zusammensetzung mit verbesserter Bioaktivität in Lebewesen, wobei die Zusammensetzung umfasst:
(a) eine antibiotische Verbindung;
(b) eine wirksame Menge Lysergol, 1-10 µg/ml; und
(c) optional pharmazeutisch verträgliche Additive.

2. Pharmazeutische Zusammensetzung nach Anspruch 1, worin das Antibiotikum ausgewählt ist aus der Gruppe, bestehend aus Rifampicin, Tetracyclin und Ampicillin.

3. Pharmazeutische Zusammensetzung nach Anspruch 1, worin die bevorzugte Dosis von Lysergoi 10 µg/ml ist.

4. Pharmazeutische Zusammensetzung nach Anspruch 1, worin die verstärkte Aktivität der antimikrobiellen Wirkung im Bereich des 2-12-fachen über einzeln verwendeter antibiotischer Verbindungen ist.

5. Pharmazeutische Zusammensetzung nach Anspruch 1, worin die Zusammensetzung wirksam ist gegen ein breites Mikrobenspektrum, sowohl gram-positiv als auch gram-negativ, ausgewählt aus der Gruppe, bestehend aus E. coli, Bacillus subtilis und Mycobacterium smegmatis und anderen ähnlichen Mikroben.

6. Pharmazeutische Zusammensetzung nach Anspruch 1, worin Lysergol isoliert ist aus Gattungen niederer Pilze, bestehend aus Claviceps, Pencillium und Rhizopus und aus den Pflanzen, ausgewählt aus Rivea corymbosa und Ipomoea violace.

7. Pharmazeutische Zusammensetzung nach Anspruch 1, worin Lysergol den Transport von Antibiotika durch den Intestinaltrakt und die Zellmembran zur besseren Effizienz an der Zielstelle verbessert.

8. Pharmazeutische Zusammensetzung nach Anspruch 1, worin die reduzierte Dosis von Antibiotika und die verbesserte Bioaktivität der Zusammensetzung die Nebenwirkungen von Antibiotika verringern.

9. Pharmazeutische Zusammensetzung nach Anspruch 1, worin die Resistenz gegenüber Antibiotikum wesentlich verringert ist aufgrund reduzierter Konzentration von Antibiotikum.

10. Pharmazeutische Zusammensetzung nach Anspruch 1, worin das Lebewesen ausgewählt ist aus Säugern und Menschen.

11. Zusammensetzung nach einem der vorhergehenden Ansprüche zur Verwendung als synergistisches Antibiotikum.

## Revendications

1. Composition pharmaceutique antibiotique synergique présentant une activité biologique accrue chez des sujets, ladite composition comprenant :
(a) un composé antibiotique ;
(b) une quantité efficace de lysergol comprise dans l'intervalle de 1 à 10 µg/ml ; et
(c) facultativement, des additifs pharmaceutiquement acceptables.

2. Composition pharmaceutique suivant la revendication 1, dans laquelle l'antibiotique est choisi dans le groupe consistant en rifampicine, tétracycline et ampicilline.

3. Composition pharmaceutique suivant la revendication 1, dans laquelle la dose préférable de lysergol est égale à 10 µg/ml.

4. Composition pharmaceutique suivant la revendication 1, dans laquelle l'activité accrue d'effet antimicrobien est de 2 à 12 fois celle des composés antibiotiques utilisés isolément.

5. Composition pharmaceutique suivant la revendication 1, qui est efficace contre des microorganismes à large spectre Gram-positifs et Gram-négatifs, choisis dans le groupe consistant en *E*. *coli, Bacillus subtilis* et *Mycobacterium smegmatis* et d'autres microorganismes similaires.

6. Composition pharmaceutique suivant la revendication 1, dans laquelle le lysergol est isolé de genres de champignons inférieurs consistant en Claviceps, Penicillium et Rhizopus et de plantes choisies entre Rivea corymbosa et Ipomoea violacea.

7. Composition pharmaceutique suivant la revendication 1, dans laquelle le lysergol accroît le transport d'antibiotiques à travers l'intestin et les membranes cellulaires pour une meilleure efficacité sur le site cible.

8. Composition pharmaceutique suivant la revendication 1, dans laquelle la dose réduite d'antibiotique et l'activité biologique accrue de la composition réduisent les effets néfastes des antibiotiques.

9. Composition pharmaceutique suivant la revendication 1, dans laquelle la résistance aux antibiotiques est substantiellement réduite à une concentration réduite en antibiotique.

10. Composition pharmaceutique suivant la revendication 1, dans laquelle le sujet est choisi entre des mammifères et l'homme.

11. Composition suivant l'une quelconque des revendications précédentes, destinée à être utilisée comme antibiotique synergique.
